# EUROPÄISCHE PATENTSCHRIFT

(11) **EP 2 976 637 B1**
(45) Veröffentlichungstag und Bekanntmachung des Hinweises auf die Patenterteilung: **05.04.2023**
(21) Anmeldenummer: 14720884.7
(22) Anmeldetag: 17.02.2014
(51) Int. Cl.: G01N 33/44, G01N 25/14

(54) **PRÜFVORRICHTUNG**
TESTING DEVICE
DISPOSITIF DE CONTRÔLE

(30) Priorität: 21.03.2013 DE 202013101214 U
(43) Veröffentlichungstag der Anmeldung: 27.01.2016
(73) Patentinhaber: Barkey GmbH & Co. KG, 33818 Leopoldshöhe (DE)
(72) Erfinder: BARKEY, Thomas, 33813 Oerlinghausen (DE)
(74) Vertreter: Ostermann, Thomas
(86) Internationale Anmeldenummer: PCT/DE2014/100060
(87) Internationale Veröffentlichungsnummer: WO 2014/146641

(56) Entgegenhaltungen:
- US-A- 4 950 608
- US-A- 5 229 580
- US-A- 5 399 840
- US-A1- 2011 165 628
- "DIN 75201:2011-11 Bestimmung des Foggingverhaltens von Werkstoffen der Kraftfahrzeug-Innenausstattung. [Determination of the fogging characteristics of trim materials in the interior of automobiles]", DEUTSCHE NORMEN. DIN NORM, BEUTH, GERMANY , Bd. DIN 75201:2011-11 1. November 2011 (2011-11-01), Seiten 1-22, XP008169716, Gefunden im Internet: URL:http://www.beuth.de/en/standard/din-75 201/144385173
- "Temperature Control Unit for the Fogging Test acc. to DIN 75201 and ISO 6452/2000", , 1. September 2001 (2001-09-01), XP055121158, Gefunden im Internet: URL:http://www.nor.com.pt/images/upload/TH ERMO_ELECTRON_Fogging-en.pdf [gefunden am 2014-06-02]

## Beschreibung

Die Erfindung betrifft eine Vorrichtung zum Prüfen von Materialeigenschaften von Materialproben nach dem Oberbegriff des Patentanspruchs 1.

Von der Firma Thermo Fisher Scientific ist unter der Produktbezeichnung "Horizon FTS" eine Vorrichtung zum Prüfen von Ausgasungen flüchtiger Bestandteile von Materialproben bekannt. Diese Geräte ermöglichten somit die Prüfung des sogenannten Foggingverhaltens von Kunststoffmaterialen, die beispielsweise im Kraftfahrzeug eingesetzt werden. Die Messung erfolgt nach der Norm DIN 75201, nach der Ausgasungen bzw. flüchtige Bestandteile von Materialproben gravimetrisch oder reflektometrisch untersucht werden. Hierzu ist es erforderlich, dass die Materialprobe über einen bestimmten Zeitraum auf eine vorgegebene Prüftemperatur aufgeheizt wird. Eine gekühlte Prüffläche sorgt dafür, dass die durch Aufheizung der Materialprobe entstehende Ausgasung durch Kondensation an der Prüffläche ansammelt. Eine zur Erwärmung zur Materialprobe vorgesehene Heizeinrichtung besteht aus einem aufgeheiztem Ölbad, das sich in einem wannenförmigen Gehäuse befindet. In der Heizflüssigkeit sind mit Ausnehmungen versehene Kolben eingetaucht, in denen jeweils die Materialproben auf einem Boden der Ausnehmung positioniert sind. Deckseitig ist die Ausnehmung von einer Prüffläche abgedeckt, die über ein externes Kühlgerät auf eine vorgegebene Temperatur gekühlt wird. An der Prüffläche bilden sich dann die Ablagerungen bzw. flüchtigen Ausgasungen, die in einem abschließenden Prüfschritt gravimetrisch oder reflektometrisch untersucht werden. Da die Heizeinrichtung ein Wärmebad aufweist, sind besondere Maßnahmen zur Erfüllung der Dichtigkeitsanforderungen erforderlich. Weiterhin muss das Wärmebad in regelmäßigen Abständen erneuert werden. Die Handhabung der Vorrichtung ist somit relativ aufwendig und kostenintensiv.

Aus der DIN 75201 ist eine Vorrichtung zum Prüfen von Materialeigenschaften von Materialproben bekannt, die ein Gehäuse zur Aufnahme von Probebehältern, eine Heizeinrichtung zur Erwärmung der Materialproben und eine gekühlte Prüffläche aufweist, so dass sich die Materialprobe ablagern kann. Die Heizeinrichtung ist als ein Wärmebad ausgebildet, wobei die Wärme mittels eines Badthermostats punktuell in dieses Wärmebad eingebracht wird.

Aus der US 5 229 580 A ist eine Vorrichtung zum Prüfen von Materialeigenschaften von Materialproben bekannt, die ein Gehäuse mit einer Anzahl von Ausnehmungen für die Probebehälter sowie einer Heizeinrichtung mit einem aufheizbaren Metallblock auf. Zur Aufheizung des Metallblocks ist eine Aufheizbohrung vorgesehen. Die Wärme wird somit linienförmig entlang der Bohrung eingebracht und nicht - wie beim Erfindungsgegenstand - flächig von einer Grundseite des Metallblocks her.

Aus US 4 950 608 A ist eine Vorrichtung zum Prüfen von Materialeigenschaften bekannt, die einen aufheizbaren Metallblock aufweist, der Ausnehmungen aufweist zur Aufnahme von Teströhrchen befinden. An einer Grundseite des Metallblocks befindet sich eine elektrisch betreibbare Heizquelle, die als Widerstandsheizung ausgebildet ist. Sie weist eine Mehrzahl von elektrischen Leitern auf, die von thermischem Isolationsmaterial umgeben sind.

Aufgabe der vorliegenden Erfindung ist es daher, eine Vorrichtung zum Prüfen von Materialeigenschaften an Materialproben derart weiterzubilden, dass die Handhabung vereinfacht und ein langzeitstabiler und zuverlässiger Betrieb der Vorrichtung gewährleistet ist.

Zur Lösung dieser Aufgabe weist die Erfindung die Merkmale des Patentanspruchs 1 auf.

Der besondere Vorteil der Erfindung besteht darin, dass eine einfache und sichere Heizeinrichtung vorgesehen ist, die insbesondere die Handhabung der Prüfvorrichtung vereinfacht. Die erfindungsgemäße Heizeinrichtung weist ein festes Wärmeübertragungsmedium, nämlich einen Metallblock auf, mittels dessen die Wärme dem Probenbehälter bzw. der in dem Probenbehälter angeordneten Materialprobe zugeführt wird. Der Metallblock ist vorzugsweise homogen erwärmt und ermöglicht eine gezielte und umfassende Wärmeübertragung bzw. Wärmeeinbringung in die Materialprobe.

Nach der Erfindung ist in einer Kühlmittelverteileinheit ein Anschluss für ein Entlüftungsventil integriert, sodass bei Inbetriebnahme oder Wechsel des Kühlmediums in den Kühlmediumkreislauf eingetretene Luft leicht entfernt werden kann. Damit die unerwünschte Luft innerhalb der Kühlmittelverteileinheit leicht entweichen kann, ist der Anschluss für das Entlüftungsventil in Höhe eines oberen Bereichs einer Kammer der Kühlmittelverteileinheit angeordnet, in der sich das zu Kühlorganen der Prüfvorrichtung zuzuführende Kühlmedium bzw. von den jeweiligen Kühlorganen kommende Kühlmedium sammelt. Vorteilhaft weist die Kühlmittelverteileinheit Kammerwände auf, die sich in vertikaler Richtung hin verjüngen, sodass sich die Luft in der Nähe des Anschlusses für das Entlüftungsventil sammelt und dann leicht entweichen kann.

Der Metallblock kann massiv aus einem Stück hergestellt sein. Alternativ kann der Metallblock auch modular aus mehreren Segmentteilen hergestellt sein, die zu dem Metallblock zusammengefügt werden.

Nach einer bevorzugten Ausführungsform der Erfindung besteht der Metallblock aus einem aluminiumbasiertem Material. Vorzugsweise handelt es sich um eine Aluminiumlegierung, so dass der volumige Metallblock relativ leicht ausgebildet ist.

Nach einer Weiterbildung der Erfindung ist der Metallblock quaderförmig ausgebildet und weist eine an die Form des Gehäuses angepasste Form auf. Der Metallblock kann das Gehäuse in dem Materialprobenbereich vollständig ausfüllen.

Nach einer Weiterbildung der Erfindung schließt sich an einer Grundseite des Metallblocks eine elektrisch betriebene Heizquelle an, wobei die Wärme flächig (d.h. homogen) in den Metallblock eingebracht wird. Dadurch, dass die Heizquelle elektrisch betrieben wird, kann die Wärmeeinbringung leicht gesteuert bzw. die Prüftemperatur einfach geregelt werden.

Nach einer Weiterbildung der Erfindung ist die Heizquelle als ein Flächenheizelement bzw. eine Heizplatte ausgeführt. Alternativ kann die Heizplatte oder der Metallblock mit Sacklöchern zur Aufnahme von Heizpatronen ausgebildet sein. Die Dimension dieser Bauteile ist an die erforderliche Heizleistung angepasst ausgebildet.

Nach einer Weiterbildung der Erfindung ist der Metallblock als ein durch Gie-ßen hergestelltes Gussteil ausgebildet. Das so hergestellte Wärmeübertragungsmittel ist somit relativ einfach herstellbar. Alternativ ist der Metallblock durch spanende Bearbeitung erstellt.

Nach einer Weiterbildung der Erfindung weist der Metallblock mindestens ein Sackloch auf, mittels dessen die Ausnehmung für die Aufnahme des Probenbehälters gewährleistet ist. Wenn der Metallblock durch Gießen hergestellt wird, kann durch entsprechende Ausbildung der Gussform das Sackloch im Rahmen des Urformvorganges entstehen.

Nach einer Weiterbildung der Erfindung weist das hohlförmige Kühlorgan eine Kammer auf, die auf einer vertikalen Oberseite dachförmig verläuft. Hierzu weist eine obere Flachseite des Kühlorgans entsprechende Dachsegmente auf. An einer obersten Kante der Dachsegmente bzw. an einem oberen Punkt des so gebildeten Daches ist ein Leitungsanschluss für eine Rücklaufleitung und eine Zulaufleitung des Kühlmediums angeordnet, sodass sich die unerwünschte Luft zielgerichtet in einem oberen Bereich der Kammer sammeln und dann über die Leitungen in Richtung der Kühlmediumverteileinheit abgeleitet werden kann, wo sie über das Entlüftungsventil nach außen abgeführt wird.

Weitere Vorteile der Erfindung ergeben sich aus den weiteren Unteransprüchen.

Ein Ausführungsbeispiel der Erfindung wird nachfolgend anhand der Zeichnungen näher erläutert.

Es zeigen:

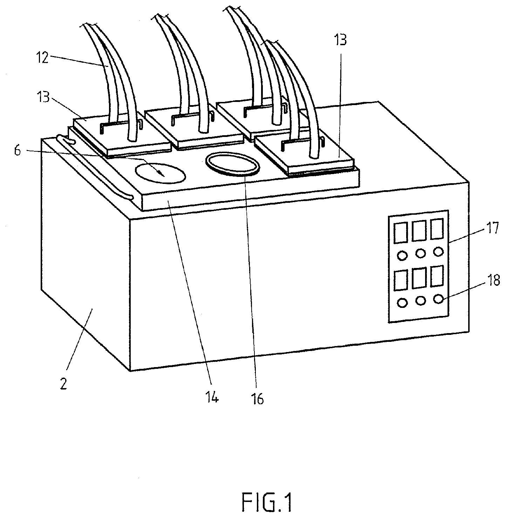

- Figur 1: eine perspektivische Darstellung einer Prüfvorrichtung mit einem Gehäuse und mehreren Probenbehältern, auf denen jeweils Kühlorgane angeordnet sind, die mit einem nicht dargestellten externen Kühlgerät gekoppelt sind,

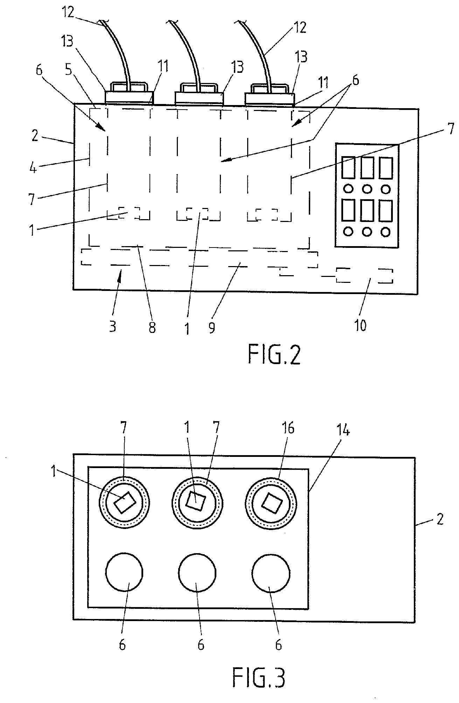

- Figur 2: eine Seitenansicht des Gehäuses und
- Figur 3: eine Draufsicht auf das Gehäuse mit entfernten Kühlorganen,
- Figur 4a: eine perspektivische Vorderansicht einer Grundplatte einer Kühlmediumverteileinheit, die an einer Rückseite der Prüfvorrichtung befestigt ist,
- Figur 4b: eine Vorderansicht der Grundplatte mit ausgeformten Kammern,
- Figur 5: eine Vorderansicht einer Abdeckplatte der Kühlmediumverteileinheit,
- Figur 6: eine perspektivische Darstellung einer oberen Flachseite des Kühlorgans von unten gesehen,

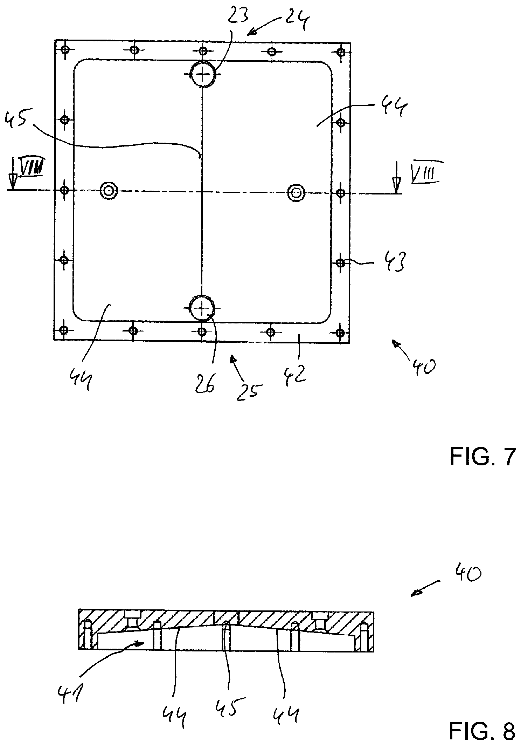

- Figur 7: eine Untenansicht der oberen Flachseite des Kühlorgans und
- Figur 8: einen Schnitt durch die obere Flachseite des Kühlorgans entlang Schnittlinie VIII-VIII in Figur 7.

Eine erfindungsgemäße Vorrichtung zum Prüfen von Materialeigenschaften an Materialproben kann insbesondere zurquantitativen Untersuchung derselben eingesetzt werden. Im vorliegenden Ausführungsbeispiel dient die Vorrichtung zum Prüfen von Ausgasungen flüchtiger Bestandteile einer Materialprobe 1, die beispielsweise von einer Inneneinrichtung eines Kraftfahrzeugs entnommen worden ist. Die Prüfvorrichtung umfasst ein quaderförmiges Gehäuse 2, in dem eine Heizeinrichtung 3 zur Erwärmung der Materialprobe1 auf eine vorgegebene Prüftemperatur angeordnet ist. Die Heizeinrichtung 3 weist als Wärmeübertragungsmittel einen Metallblock 4 auf, der an einer Oberseite 5 eine Anzahl von Ausnehmungen 6 aufweist. Im vorliegenden Ausführungsbeispiel weist der vorzugsweise massiv ausgebildete Metallblock 4 sechs Ausnehmungen auf, in die jeweils Probenbehälter 7 einführbar sind. Die Probenbehälter 7 sind als zylinderförmige Glasbehälter ausgebildet, deren Außendurchmesser im Wesentlichen dem Innendurchmesser der Ausnehmung 6 entspricht, so dass die Probenbehälter 7 unter Spielpassung in die jeweiligen Ausnehmungen 6 eingeschoben werden können. Auf einem Boden der Probenbehälter 7 sind die Materialproben 1 positioniert.

Der Metallblock 4 ist vorzugsweise aus einem aluminiumbasiertem Material hergestellt, also einer Aluminiumlegierung oder einem anderen thermisch leitenden Material.

Ferner umfasst die Heizeinrichtung 3 eine vorzugsweise elektrisch betreibbare Heizquelle, die sich an einer Grundseite 8 des Metallblocks 4 anschließt, wobei sie vorzugsweise an der Grundseite 8 anliegt, so dass durch die Heizquelle erzeugte Wärme flächig (d.h. homogen) in den Metallblock eingebracht wird. Die Heizquelle ist als eine Heizplatte 9 bzw. als ein Flächenheizelement ausgebildet, das nach Art einer Widerstandsheizung elektrische Energie in thermische Energie umwandelt. Der Metallblock 4 dient somit als Wärmeübertragungsmittel.

Alternativ kann die Heizplatte auch Sacklöcher aufweisen zur Aufnahme von Heizpatronen.

Die Heizplatte 9 oder die alternativen Heizungsausführungen sind über ein Kabel mit einer elektrischen Steuereinheit 10 gekoppelt, mittels derer ein von der erforderlichen Heizleistung abhängiger elektrischer Strom in die Heizplatte 9 eingeprägt wird. Vorzugsweise weist die Steuereinheit 10 ein Regelmodul auf, mittels dessen die Prüftemperatur auf eine vorgegebene Solltemperatur geregelt wird.

Alternativ können in dem Metallblock 4 Heizelemente integriert sein, so dass der Wärmeübertragungsweg zu den Probebehältern verringert ist. Beispielsweise kann der Metallblock 4 Sacklöcher aufweisen zur Aufnahme von Heizpatronen oder Flächenheizungen, welche am oder im Heizblock integriert werden.

Der Metallblock 4 kann beispielsweise durch Gießen hergestellt sein, wobei ein Gussteil mit den entsprechenden Ausnehmungen 6 geformt worden ist.

Alternativ kann der Metallblock 4 aus mehreren Segmentteilen bestehen, die in dem Gehäuse 2 aufeinander geschichtet bzw. zusammengesetzt werden. Der Metallblock kann, statt als ein einstückiges massives Wärmeübertragungsmittel ausgebildet zu sein, auch modular aus mehreren Segmentteilen hergestellt sein, die zu dem Metallblock zusammengefügt werden.

Alternativ kann der Metallblock 4 auch aus jeweils den Probenbehältern 7 zugeordnete Metallsegmente aufweisen, die jeweils die Ausnehmungen aufweisen und bei denen benachbarte Metallsegmente unmittelbar aneinander liegen oder beabstandet zueinander angeordnet sind.

Damit sich die Ablagerungen der Materialprobe 1 an einer Prüffläche 11 durch Kondensieren sammeln können, ist ein nicht dargestelltes externes Kühlgerät über Leitungen bzw. Schläuche 12 mit Kühlorganen 13 verbunden, die flächig auf der Prüffläche 11 aufliegen. Wird ein reflektometrisches Messverfahren eingesetzt, ist die Prüffläche 11 als eine Glasplatte ausgebildet, die die obere Öffnung des Probenbehälters 7 abschließt. Dadurch, dass die Glasplatte 11 von den Kühlorganen 13 gekühlt wird, setzen sich auf einer der Ausnehmung 6 zugewandten Flachseite der Glasplatte 11 Ablagerungen der Materialprobe 1 ab bzw. kondensieren ausgegaste flüchtige Bestandteile der Materialprobe 1.

Über die Schläuche 12, die als eine Zulaufleitung und eine Rücklaufleitung dienen, wird in die Kühlorgane 13 ein Kühlmedium ein- bzw. ausgeleitet, so dass die Prüffläche 11 die für die Kondensation der Ausgasung erforderliche Kühltemperatur von bspw. 20°C aufweist.

Damit die Glasbehälter 7 eine definierte Lage einnehmen und besser transportiert werden können, ist ein tragbarer Rahmen 14 mit Aufnahmeöffnungen 15 vorgesehen, deren lichte Weite jeweils größer ist als ein Durchmesser der Glasbehälter 7 einerseits und kleiner als ein oberer Öffnungsrand 16 des Glasbehälters 7 andererseits. Auf diese Weise ist der Glasbehälter 7 sicher in der Aufnahmeöffnung 15 des Rahmens 14 gehalten.

An einer Vorderseite des Gehäuses 2 sind ein Display 17 sowie Tasten 18 angeordnet, so dass eine Bedienperson die gewünschte Heiztemperatur, beispielsweise 100°C, und ggf. die Heizdauer, beispielsweise 3 bis 6 Stunden, einstellen kann. Die Steuereinheit 10 ist vorzugsweise so ausgelegt, dass die Heizplatte 9 auf eine bestimmte Temperatur geregelt wird, so dass stets ein gleicher Wärmeeintrag in die jeweiligen Ausnehmungen 6 gewährleistet ist.

Nachdem die Materialprobe 1 in dem Prüfzeitraum auf die vorgegebene Prüftemperatur gehalten worden ist, wird die Heizeinrichtung 3 bzw. das Kühlgerät abgeschaltet. Anschließend werden die Kühlorgane 13 entfernt, so dass nun die sich an der gekühlten Prüffläche 11 niedergeschlagenen Ablagerungen entsprechend der Norm DIN 75201 bestimmt werden können. Hierzu weist eine nicht dargestellte Messeinrichtung Mittel zur reflektometrischen Messung auf. Hierbei wird die Reflexion der niedergeschlagenen Ablagerung in bekannter Weise bestimmt.

Soll zur Foggingprüfung ein gravimetrisches Messverfahren eingesetzt werden, ist die Prüffläche als eine Folie ausgebildet, an der sich die Ablagerungen sammeln. Mit Hilfe einer gravimetrischen Waage kann dann quantitativ die Stoffmenge der Ablagerung ermittelt werden.

Soll eine Wasserdampf-Diffusionsprüfung vorgenommen werden, werden einseitig beschichtete Probenbleche auf die mit einer Probesubstanz gefüllten Probenbehälter gelegt. Auf die nichtbeschichtete Seite wird das Kühlorgan 13 gesetzt. Durch Kondensation bzw. Bläschenbildung an der beschichteten Seite kann auf wasserdampfdichtes Beschichtungsmaterial geschlossen werden. Dieses Prüfverfahren kann für viele Korrosionsprüfverfahren im analytischen Bereich eingesetzt werden.

Von dem Gehäuse 2 erhebt sich ein nicht dargestelltes Gestell, das rahmenförmig ausgebildet ist mit mindestens einer waagerechten Haltestange, an der die Kühlorgane 13 in einer Nichtgebrauchsstellung befestigt, abgelegt oder eingehängt werden können. Aufgrund der sicheren Lagerung der Kühlorgane 13 an dem Gestell und des ergonomisch kurzen Weges von der Befestigungsstelle am Gestell zur Abdecklage am oberen Randbereich der Probenbehälter 7 kann die Handhabung der Prüfvorrichtung verbessert werden.

Der Rahmen 14 dient als Zentrierrahmen zur passgenauen Lagerung der Kühlorgane 13 in der Gebrauchsstellung derselben.

Eine Kühleinrichtung besteht im Wesentlichen aus dem externen Kühlgerät, kühlmediumführenden Leitungen, einer Kühlmediumverteileinheit 20 sowie den Kühlorganen 13. Das externe Kühlgerät kühlt das Kühlmedium auf eine vorgegebene Temperatur und leitet dieses mittels einer integrierten Pumpe über eine nicht dargestellte Zulaufleitung an einen eingangsseitigen Leitungsanschluss 21 der Kühlmediumverteileinheit 20. Die Kühlmediumverteileinheit 20 verteilt das zugeleitete Kühlmedium über Leitungsanschlüsse 22 an den jeweiligen Leitungsanschluss 23 der Kühlorgane 13. Der eingangsseitige Leitungsanschluss 23 befindet sich an einem ersten Ende 24 des Kühlorgans 13. An einem gegenüberliegenden zweiten Ende 25 des Kühlorgans 13 ist ein Leitungsanschluss 26 angeordnet, von dem aus über die kühlmittelführenden Leitungen (Schläuche 12) das Kühlmedium an entsprechende Leitungsanschlüsse 27 der Kühlmediumverteileinheit 20 zurückgeleitet wird.

Das von den jeweiligen Kühlorganen 13 kommende Kühlmedium sammelt sich in einer Ausgangskammer 28 der Kühlmediumverteileinheit 20, von wo aus es über einen ausgangsseitigen Leitungsanschluss 29 über nicht dargestellte Leitungen an das externe Kühlgerät weitergeleitet wird. Im Betrieb der Prüfvorrichtung erfolgt ein kontinuierliches Umwälzen des Kühlmediums zwischen dem externen Kühlgerät auf der einen Seite und den Kühlorganen 13 auf der anderen Seite.

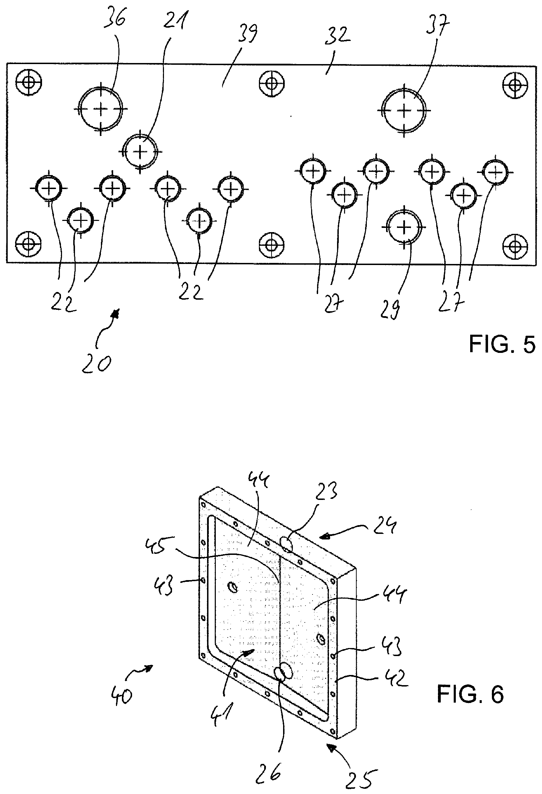

Die Kühlmediumverteileinheit 20 weist ferner eine Eingangskammer 30 auf, in der das über den eingangsseitigen Leitungsanschluss 21 zugeführte Kühlmedium eingeleitet und über die mehreren Leitungsanschlüsse 22 zu den jeweiligen Kühlorganen 13 verteilt wird. Aus Figur 5 ist ersichtlich, dass über die Leitungsanschlüsse 22 sechs Zulaufleitungen an die Kühlorgane 13 und über die Leitungsanschlüsse 27 sechs Rücklaufleitungen zu den sechs Kühlorganen 13 verlaufen, wobei jedes Kühlorgan 13 über den eingangsseitigen Leitungsanschluss 23 und dem Schlauch mit einem der Leitungsanschlüsse 22 und über den weiteren Leitungsanschluss 26 über den Schlauch 12 mit einem der Leitungsanschlüsse 27 der Kühlmediumverteileinheit 20 verbunden ist.

Die Kühlmediumverteileinheit 20 weist eine Grundplatte 31 mit der ausgeformten Eingangskammer 30 und der ausgeformten Ausgangskammer 28 auf. Die Grundplatte 31 ist über Befestigungsmittel mit einer Abdeckplatte 32 verbunden, die den Leitungsanschluss 21 für die von dem externen Kühlgerät kommende Zulaufleitung sowie die Leitungsanschlüsse 22 für die zu den Kühlorganen 13 führenden Zulaufleitungen 12 auf einer Eingangsseite und die den Leitungsanschluss 29 für die zu dem externen Kühlgerät verlaufende Rücklaufleitung sowie die Leitungsanschlüsse 27 für die von den jeweiligen Kühlorganen 13 verlaufenden Rücklaufleitungen 12 auf einer Ausgangsseite aufweist.

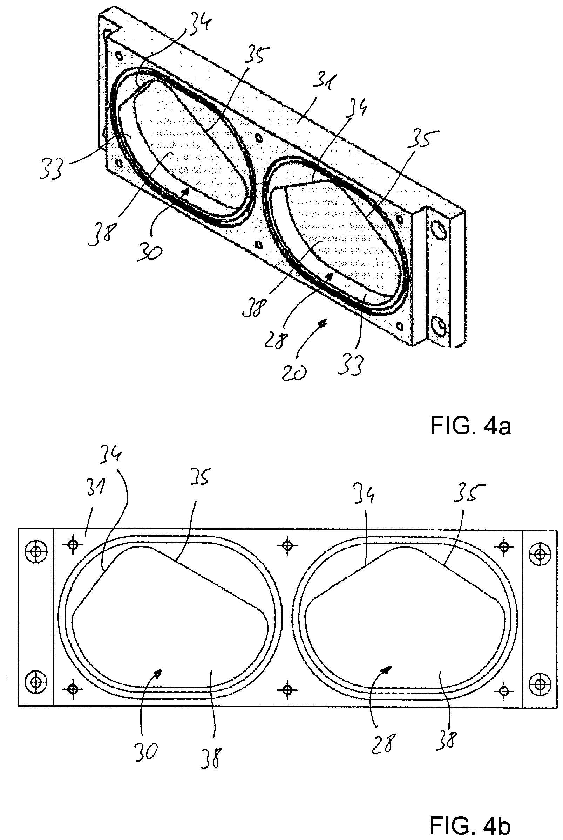

Aus den Figuren 4a und 4b ist ersichtlich, dass die Eingangskammer 30 und die Ausgangskammer 28 jeweils durch gegenüberliegende Flachseiten 38 der Grundplatte 31 und einer Flachseite 39 der Abdeckplatte 32 und einer Schmalseite 33 begrenzt ist. Die Schmalseite 33 ist in der Platte 31 integriert, wobei sich im Bereich der Eingangs-und Ausgangskammer topfförmige Ausnehmungen bilden. Die Schmalseiten 33 der Eingangskammer 30 und der Ausgangskammer 28 weisen jeweils sich in vertikaler Richtung nach oben verjüngende Wände 34 und 35 auf, die in einer Höhe zusammenlaufen, in der ein der Eingangskammer zugeordneter Anschluss 36 und ein der Ausgangskammer 28 zugeordneter Anschluss 37 für ein Entlüftungsventil an der Abdeckplatte 32 vorgesehen ist.

Wie insbesondere aus Figur 5 ersichtlich ist, sind die Anschlüsse 36, 37 für die entsprechenden Entlüftungsventile sind oberhalb der zu der Eingangskammer 30 und der Ausgangskammer 28 führenden Leitungsanschlüsse 21, 29 sowie 22 und 27 angeordnet, sodass bei einer Inbetriebnahme der Prüfvorrichtung oder einem Wechsel des Kühlmediums auf einfache und schnelle Weise Luft aus dem Leitungssystem entweichen kann.

Das Kühlorgan 13 ist hohlförmig ausgebildet und weist eine obere Flachseite 40 und eine nicht dargestellte untere Flachseite 40 auf, die plattenförmig ausgebildet ist und mit der oberen Flachseite 40 fest verbunden ist zur Bildung einer Kammer 41. Die obere Flachseite 40 weist einen umlaufenden Rand 42 auf, der Bohrungen 43 zur Befestigung mit der nicht dargestellten unteren Flachseite aufweist. Ferner begrenzt der Rand 42 die Kammer 41 von der Seite her. Von einer in vertikaler Richtung oberen Seite wird die Kammer 41 durch die obere Flachseite 40 begrenzt, die aus zwei Dachsegmenten 44 besteht, die sich dachförmig unter einem geneigten Winkel zu einer obersten Kante 45 der oberen Flachseite 40 hin erheben. Die Dachsegmente 44 erstrecken sich unter einem spitzen Winkel zu einer Ebene, die durch die umlaufende Randfläche 42 gebildet ist. Vorzugsweise liegt der Neigungswinkel in einem Bereich zwischen 10° und 30 °. An der oberen Kante 45 und zwar an dem ersten Ende 24 und an dem gegenüberliegenden zweiten Ende 25 des Kühlorgans 13 befinden sich die Leitungsanschlüsse 23, 26 für die Zulaufleitung und die Rücklaufleitung des Kühlmediums. Die Leitungsanschlüsse 23, 26 befinden sich somit an einem höchsten Punkt der Kammer 41, sodass bei Inbetriebnahme oder bei Wechsel des Kühlmediums relativ schnell und einfach Luft in die Leitungen 12 entweichen kann. Nachdem die Luft über die Schläuche 12 in die Eingangskammer 30 bzw. in die Ausgangskammer 28 geleitet worden ist, kann sie über die an den Anschlüssen 36, 37 angeordneten Entlüftungsventilen aus dem Kühlmediumkreislauf entfernt werden. Es ist ersichtlich, dass die Entlüftung an den höchsten Punkten der jeweiligen Kammer 41 bzw. der Eingangskammer 30 bzw. der Ausgangskammer 28 erfolgt.

Alternativ kann bei größerer Dimensionierung der Kammern der Anschluss auch in einer horizontalen Ebene angeordnet sein, die den höchsten Punkt der Kammer schneidet. Auf diese Weise ist stets gewährleistet, dass Luft mit geringem Aufwand aus dem Kühlmittelkreislauf entfernt werden kann.

Die Wände der Kammern enden an einem höchsten Punkt der Kammer, in dessen horizontaler Ebene der Anschluss angeordnet ist.

Nach einer alternativen Ausführungsform der Erfindung kann die Kammer lediglich eine einzige unter einem Winkel in vertikaler Richtung nach oben verlaufende Wand aufweisen, dessen Ende mit einem oberen Ende einer vertikalen Wand zusammenfällt. Wenn im Bereich dieses oberen Endes der Anschluss vorgesehen ist, kann ebenfalls die Luft leicht abgeführt werden.

## Patentansprüche

1. Vorrichtung zum Prüfen von Materialeigenschaften von Materialproben (1), insbesondere zum Prüfen von Ausgasungen flüchtiger Bestandteile einer Materialprobe (1),
- mit einem Gehäuse enthaltend eine Anzahl von Ausnehmungen zur Aufnahme von einem Materialprobe (1) enthaltenen Probebehälter (7),
- mit einer Heizeinrichtung (3) zur Erwärmung der Materialprobe (1) auf eine vorgegebene Prüftemperatur,
- mit einer den Probebehälter (7) abdeckenden und auf eine Kühltemperatur gekühlten Prüffläche (11), so dass sich an der Prüffläche (11) eine Ablagerung der Materialprobe (1) bildet, **dadurch gekennzeichnet, dass** die Heizeinrichtung (3) einen aufheizbaren massiven oder modularen Metallblock (4) aufweist, der die Ausnehmung (6) mit Ausnahme eines Öffnungsbereichs vollständig umgibt,
- mit einem externen Kühlgerät, das über Kühlmittel führende Leitungen mit einer Kühlmediumverteileinheit (20) verbunden ist, wobei die Kühlmediumverteileinheit (20) jeweils über eine Zulaufleitung (12) und eine Rücklaufleitung mit Kühlorganen (13) gekoppelt ist und wobei die Prüffläche (11) mit dem von dem Kühlmedium durchströmten Kühlorgan (13) gekoppelt ist zur Kühlung derselben auf eine vorgegebene Temperatur,
- dass die Kühlmediumverteileinheit (20) eine Grundplatte (31), eine Abdeckplatte (32) und Schmalseiten (33) aufweist zur Bildung einer Eingangskammer (30) für den Zulauf des von dem externen Kühlgerät kommenden Kühlmediums und zur Bildung einer Ausgangskammer (20) für den Rücklauf des von dem Kühlorgan (13) kommenden Kühlmediums, wobei die Abdeckplatte (32) einen der Eingangskammer (30) zugeordneten Leitungsanschluss (21) für die von dem externen Kühlgerät kommende Zulaufleitung und der Eingangskammer (30) zugeordneten Leitungsanschlüsse (22) für die zu den Kühlorganen (13) führenden Zulaufleitungen (12) einerseits und eine der Ausgangskammer (20) zugeordneten Leitungsanschluss (29) für die zu dem externen Kühlgerät verlaufende Rücklaufleitung und der Ausgangskammer (20) zugeordneten Leitungsanschlüssen (27) für die von den jeweiligen Kühlorganen (13) führenden Rücklaufleitungen andererseits aufweist, wobei an den Schmalseiten (33) der Eingangskammer (30) und der Ausgangskammer (28) in vertikaler Richtung nach oben verjüngende Wände (34, 35) vorgesehen sind, die in Höhe eines Anschlusses (36, 37) für das Entlüftungsventil zusammenlaufen.

2. Prüfvorrichtung nach Anspruch 1, **dadurch gekennzeichnet, dass** der Metallblock (4) aus einem aluminiumbasierten Material besteht.

3. Prüfvorrichtung nach Anspruch 1 oder 2, **dadurch gekennzeichnet, dass** der Metallblock (4) quaderförmig ausgebildet ist.

4. Prüfvorrichtung nach einem der Ansprüche 1 bis 3, **dadurch gekennzeichnet, dass** sich an eine Grundseite (8) des Metallblocks (4) eine elektrisch betreibbare Heizquelle (9) unter flächiger Anlage an der Grundseite (8) anschließt.

5. Prüfvorrichtung nach Anspruch 4, **dadurch gekennzeichnet, dass** die Heizquelle (9) als ein Flächenheizelement ausgebildet ist oder dass der Metallblock (4) oder die Heizquelle (9) Sacklöcher zur Aufnahme von Heizpatronen aufweist oder dass Flächenheizungen an oder im Metallblock integriert sind.

6. Prüfvorrichtung nach einem der Ansprüche 1 bis 5, **dadurch gekennzeichnet, dass** der Metallblock (4) als ein durch Gießen hergestelltes Gussteil oder als ein durch mehrere metallische Segmentteile zusammengesetzter Körper ausgebildet ist.

7. Prüfvorrichtung nach einem der Ansprüche 1 bis 6, **dadurch gekennzeichnet, dass** die Heizeinrichtung (3) eine elektrische Steuereinheit (10) aufweist zur Ansteuerung oder Regelung der Heizplatte (9) auf eine vorgegebene Temperatur.

8. Prüfvorrichtung nach einem der Ansprüche 1 bis 7, **dadurch gekennzeichnet, dass** die Ausnehmung (6) als ein Sackloch ausgebildet ist mit einem auf den Probenbehälter angepassten Durchmesser, so dass der Probenbehälter (7) unter Spielpassung in dem Sackloch gelagert ist.

9. Prüfvorrichtung nach einem der Ansprüche 1 bis 8, **dadurch gekennzeichnet, dass** eine Messeinrichtung zur quantitativen oder qualitativen Untersuchung der Ablagerung vorgesehen ist, die insbesondere zur Bestimmung des Foggingverhaltens der Materialprobe Mittel zur gravimetrischen oder reflektometrischen Messung der Ablagerung aufweist.

10. Prüfvorrichtung nach einem der Ansprüche 1 bis 9, **dadurch gekennzeichnet, dass** der Anschluss (23, 26, 36, 37) an einem in vertikaler Richtung höchsten Punkt der Kammer (28, 30, 41) oder in einer den höchsten Punkt der Kammer(28, 30, 41) schneidenden horizontalen Ebene angeordnet ist.

11. Prüfvorrichtung nach einem der Ansprüche 1 bis 10, **dadurch gekennzeichnet, dass** das Kühlorgan (13) hohlförmig ausgebildet ist mit einer oberen Flachseite (40) und mit einer unteren Flachseite, die über einen schmalseitigen Rand (42) miteinander verbunden sind, wobei innerhalb des Kühlorgans (13) die Kammer (41) angeordnet ist, und dass die obere Flachseite eine Anzahl von sich dachförmig von der unteren Flachseite erhebende Dachsegmente (44) aufweist, an deren obersten Kante (45) der Leitungsanschluss (23, 26) für die Zulaufleitung und die Rücklaufleitung angeordnet sind.

12. Prüfvorrichtung nach einem der Ansprüche 1 bis 11, **dadurch gekennzeichnet, dass** der Anschluss (36, 37) für das Entlüftungsventil in vertikaler Richtung oberhalb der Leitungsanschlüsse (21, 22, 27, 29) der Kühlmediumverteileinheit (20) angeordnet ist.

## Claims

1. A device for testing material properties of material samples (1), in particular for testing outgassing of volatile constituents of a material sample (1),
- having a housing containing a number of recesses for accommodating a sample container (7) containing material sample (1),
- having a heating apparatus (3) for heating the material sample (1) to a predetermined test temperature,
- having a testing surface (11) covering the sample container (7) and cooled to a cooling temperature, such that a deposit of the material sample (1) forms on the testing surface (11), **characterized in that** the heating apparatus (3) has a heatable solid or modular metal block (4), which completely surrounds the recess (6) except for an opening region,
- having an external cooling device, which is connected to a cooling medium distributor unit (20) via lines conveying coolant, wherein the cooling medium distributor unit (20) is in each case coupled via a feed line (12) and a return line to cooling members (13) and wherein the testing surface (11) is coupled to the cooling member (13) through which the cooling medium flows for cooling thereof to a predetermined temperature,
- **in that** the cooling medium distributor unit (20) has a base plate (31), a cover plate (32) and narrow sides (33) for forming an inlet chamber (30) for feed of the cooling medium coming from the external cooling device and for forming an outlet chamber (20) for return of the cooling medium coming from the cooling member (13), wherein the cover plate (32) has a line port (21) associated with the inlet chamber (30) for the feed line coming from the external cooling device and line ports (22) associated with the inlet chamber (30) for the feed lines (12) leading to the cooling members (13) on the one hand and a line port (29) associated with the outlet chamber (20) for the return line extending to the external cooling device and line ports (27) associated with the outlet chamber (20) for the return lines leading from the respective cooling members (13) on the other hand, wherein on the narrow sides (33) of the inlet chamber (30) and the outlet chamber (28) walls (34, 35) tapering vertically upwards are provided which converge at the level of a port (36, 37) for the vent valve.

2. The testing device according to Claim 1, **characterized in that** the metal block (4) consists of an aluminum-based material.

3. The testing device according to Claim 1 or 2, **characterized in that** the metal block (4) is formed to be cuboid-shaped.

4. The testing device according to any one of Claims 1 to 3, **characterized in that** an electrically operable heat source (9) adjoins a base side (8) of the metal block (4), resting flat against the base side (8).

5. The testing device according to Claim 4, **characterized in that** the heat source (9) is formed as a panel heating element or **in that** the metal block (4) or the heat source (9) has blind holes for accommodating cartridge heaters or **in that** panel heaters are incorporated onto or into the metal block.

6. The testing device according to any one of Claims 1 to 5, **characterized in that** the metal block (4) is formed as a cast part produced by casting or as a body composed of a plurality of metallic segment parts.

7. The testing device according to any one of Claims 1 to 6, **characterized in that** the heating apparatus (3) has an electrical control unit (10) for controlling or adjusting the hot plate (9) to a predetermined temperature.

8. The testing device according to any one of Claims 1 to 7, **characterized in that** the recess (6) is formed as a blind hole with a diameter adapted to the sample container, such that the sample container (7) is mounted in the blind hole with a clearance fit.

9. The testing device according to any one of Claims 1 to 8, **characterized in that** a measurement device for quantitative or qualitative investigation of the deposit is provided, which has means for gravimetric or reflectometric measurement of the deposit in particular to determine the fogging behavior of the material sample.

10. The testing device according to any one of Claims 1 to 9, **characterized in that** the port (23, 26, 36, 37) is arranged at a highest point, in the vertical direction, of the chambers (28, 30, 41) or in a horizontal plane intersecting with the highest point of the chambers (28, 30, 41).

11. The testing device according to any one of Claims 1 to 10, **characterized in that** the cooling member (13) is formed to be hollow, with an upper flat side (40) and with a lower flat side, which are connected together via a narrow-side edge (42), wherein the chamber (41) is arranged within the cooling member (13), and **in that** the upper flat side has a number of roof segments (44) rising in the form of a roof from the lower flat side, at the uppermost edge (45) of which roof segments the line ports (23, 26) for the feed line and the return line are arranged.

12. The testing device according to any one of Claims 1 to 11, **characterized in that** the port (36, 37) for the vent valve is arranged vertically above the line ports (21, 22, 27, 29) of the cooling medium distributor unit (20).

## Revendications

1. Dispositif pour contrôler les propriétés matérielles d'échantillons de matériau (1), en particulier pour contrôler les dégagements gazeux de constituants volatils d'un échantillon de matériau (1),
- comportant un boîtier contenant un certain nombre d'évidements pour recevoir un récipient à échantillon (7) contenant un échantillon de matériau (1),
- comportant un dispositif de chauffage (3) pour chauffer l'échantillon de matériau (1) à une température de contrôle prédéfinie,
- comportant une surface de contrôle (11) recouvrant le récipient à échantillons (7) et refroidie à une température de refroidissement, de telle sorte qu'un dépôt de l'échantillon de matériau (1) se forme sur la surface de contrôle (11), **caractérisé en ce que** le dispositif de chauffage (3) présente un bloc métallique (4) massif ou modulaire apte à être chauffé, qui entoure entièrement l'évidement (6) à l'exception d'une zone d'ouverture,
- comportant un appareil réfrigérant externe, qui est relié à une unité de distribution de fluide de refroidissement (20) par le biais de conduites véhiculant du produit de refroidissement, l'unité de distribution de fluide de refroidissement (20) étant couplée à des organes de refroidissement (13) respectivement par le biais d'une conduite d'arrivée (12) et d'une conduite de retour et la surface de contrôle (11) étant couplée à l'organe de refroidissement (13) dans lequel circule de fluide de refroidissement pour refroidir celle-ci à une température prédéfinie,
- **en ce que** l'unité de distribution de fluide de refroidissement (20) présente une plaque de base (31), une plaque de recouvrement (32) et des côtés étroits (33) pour former une chambre d'entrée (30) pour l'arrivée du fluide de refroidissement venant de l'appareil réfrigérant externe et pour former une chambre de sortie (20) pour le retour du fluide de refroidissement venant de l'organe de refroidissement (13), la plaque de recouvrement (32) présentant, d'une part, un raccord de conduite (21) associé à la chambre d'entrée (30) pour la conduite d'arrivée venant de l'appareil réfrigérant externe et des raccords de conduite (22) associés à la chambre d'entrée (30) pour les conduites d'arrivée (12) conduisant aux organes de refroidissement (13) et, d'autre part, un raccord de conduite (29) associé à la chambre de sortie (20) pour la conduite de retour s'étendant jusqu'à l'appareil réfrigérant externe et des raccords de conduite (27) associés à la chambre de sortie (20) pour les conduites de retour partant des organes de refroidissement (13) respectifs, des parois (34, 35) s'amincissant dans la direction verticale vers le haut étant prévues sur les côtés étroits (33) de la chambre d'entrée (30) et de la chambre de sortie (28), lesdites parois se rejoignant à la hauteur d'un raccord (36, 37) pour la soupape de dégagement d'air.

2. Dispositif de contrôle selon la revendication 1, **caractérisé en ce que** le bloc métallique (4) est constitué d'un matériau à base d'aluminium.

3. Dispositif de contrôle selon la revendication 1 ou 2, **caractérisé en ce que** le bloc métallique (4) est réalisé en forme de parallélépipède.

4. Dispositif de contrôle selon une des revendications 1 à 3, **caractérisé en ce qu'**une source de chauffage (9) à fonctionnement électrique est raccordée à un côté de base (8) du bloc métallique (4) en s'appliquant à plat contre le côté de base (8).

5. Dispositif de contrôle selon la revendication 4, **caractérisé en ce que** la source de chauffage (9) est réalisée sous la forme d'un élément chauffant plat ou **en ce que** le bloc métallique (4) ou la source de chauffage (9) présente des trous borgnes pour recevoir des cartouches de chauffage ou **en ce que** des éléments de chauffage par rayonnement dans la surface sont intégrés sur ou dans le bloc métallique.

6. Dispositif de contrôle selon une des revendications 1 à 5, **caractérisé en ce que** le bloc métallique (4) est réalisé sous la forme d'une pièce moulée fabriquée par coulage ou sous la forme d'un corps constitué par plusieurs segments métalliques.

7. Dispositif de contrôle selon une des revendications 1 à 6, **caractérisé en ce que** le dispositif de chauffage (3) présente une unité de commande électrique (10) pour la commande ou la régulation de la plaque de chauffage (9) à une température prédéfinie.

8. Dispositif de contrôle selon une des revendications 1 à 7, **caractérisé en ce que** l'évidement (6) est réalisé sous la forme d'un trou borgne avec un diamètre adapté au récipient à échantillon, de telle sorte que le récipient à échantillon (7) est logé dans le trou borgne par ajustement avec jeu.

9. Dispositif de contrôle selon une des revendications 1 à 8, **caractérisé en ce qu'**un dispositif de mesure est prévu pour l'analyse quantitative ou qualitative du dépôt, ledit dispositif de mesure présentant en particulier des moyens de mesure gravimétrique ou réflectométrique du dépôt pour déterminer l'effet de fogging de l'échantillon de matériau.

10. Dispositif de contrôle selon une des revendications 1 à 9, **caractérisé en ce que** le raccord (23, 26, 36, 37) est disposé au point le plus haut dans la direction verticale de la chambre (28, 30, 41) ou dans un plan horizontal passant par le point le plus haut de la chambre (28, 30, 41).

11. Dispositif de contrôle selon une des revendications 1 à 10, **caractérisé en ce que** l'organe de refroidissement (13) est réalisé de forme creuse avec un côté plat (40) supérieur et un côté plat inférieur, qui sont reliés l'un à l'autre par un bord à côté étroit (42), la chambre (41) étant disposée dans l'organe de refroidissement (13), et **en ce que** le côté plat supérieur présente un certain nombre de segments de toit (44) s'élevant en forme de toit depuis le côté plat inférieur, le raccord de conduite (23, 26) pour la conduite d'arrivée et la conduite de retour étant disposé sur l'arête (45) la plus haute de ceux-ci.

12. Dispositif de contrôle selon une des revendications 1 à 11, **caractérisé en ce que** le raccord (36, 37) pour la soupape de dégagement d'air est disposé dans la direction verticale au-dessus des raccords de conduite (21, 22, 27, 29) de l'unité de distribution de fluide de refroidissement (20).
